# EUROPEAN PATENT APPLICATION

(11) **EP 2 639 584 A1**
(43) Date of publication of application: **18.09.2013**
(21) Application number: 12001814.8
(22) Date of filing: 16.03.2012
(51) Int. Cl.: G01N 33/543

(54) **Real time diagnostic assays using an evanescence biosensor**

(71) Applicant: Davos Diagnostics AG, 7270 Davos (CH)
(72) Inventor: Schawaller, Manfred, 1785 Cressier (CH); Rhyner, Claudio, 7270 Davos (CH); Akdis, Cezmi, 7265 Davos (CH); Wiki, Max, 1110 Morges (CH); Crameri, Reto, 7270 Davos (CH)
(74) Representative: Müller-Boré & Partner Patentanwälte

(57) **Abstract**

The present invention relates to a method for the detection of a substance in an aqueous, physiological or chemical liquid using the evanescence field method, and to a diagnostic device for carrying out said method.

## Description

The present invention relates to a method for the detection of a substance in an aqueous, physiological or chemical liquid using the evanescence field method, and to a diagnostic device for carrying out said method.

Examination of blood or serum for specific biomarkers is an often used method of *in vitro* diagnostics (IVD). A blood sample is taken from the patient and centrifuged to separate the cells from the plasma. Plasma or serum are used to perform an *in-vitro* diagnostic assay for biomarkers. Although blood plasma is often used, other body fluids such as e. g. urine, saliva, cerebrospinal fluid can be tested with *in-vitro* diagnostics. Another segment of diagnostics examines cells such as erythrocytes, leukocytes and thrombocytes from blood or other cells obtained from either body fluids or other biological specimens, such as e.g. biopsies.

There are numerous biomarkers found in blood plasma indicative for infectious diseases, antibody responses, hormonal status, metabolic disorders, neoplastic syndromes, or to monitor disease states in general. Depending on the nature of the biomarker, the diagnostic assay measures qualitatively the presence or absence of a biomolecule or it measures a biomolecule quantitatively. The biomolecules can belog to many chemical or biochemical subgroups such as for example proteins, glycoproteins, DNA or RNA, lipids, saccharides or low molecular weight chemicals occurring as metabolic products. Furthermore the detection of cells, parasites, viruses, bacteria, yeast, fungi, or fragments of those organisms is also of diagnostic interest.

Markers can be molecules such as proteins, posttranslationally modified gene products, hormones, lipids, small molecules with a function in the blood or plasma, or degradation products indicative for a disease such as myelin or phenylketone. Examples of hormones determined using *in-vitro* diagnostics are the thyroid hormones or the pregnancy tests detecting human chorionic gonadotropin. The presence of the marker and/or the concentration of a hormone provides to the medical doctor valuable information supporting and guiding his diagnosis.

Markers can also be molecules with known specific function normally not present in blood, but newly appearing during a clinical disease state. A specific tissue, when damaged, can release cellular proteins from the tissue into the blood stream and proteins that are normally not detected can be found in the plasma of the patient. Examples here are the cardiac troponins found in blood, after heart muscle cell damage has taken place through a heart attack and concomitantly the myoglobin levels are elevated over the normal range. For stroke patients, proteins specific for neuronal cells appearing in the blood stream, e.g. S100 or GFAP are observed in the blood stream, after the event.

Another group of biomarkers are infectious agents, extracted and measured with specific assays. *In-vitro* diagnostics tests are detecting and measuring the infectious agent itself or fragments and components derived from these infectious agents. Infectious agents can be for example bacteria, virus, parasites, fungi, mycoplasms, or yeasts. Sometimes, the tests are performed biologically by culture or co-culture with target cells, but often *in-vitro* diagnostic tests measure pathogen-specific biochemical molecules such as proteins, DNA, RNA, oligosaccharides, lipids or glycolipids.

Immunoglobulins are a further class of molecules that are examined frequently to support diagnosis. Immunoglobulins can be markers for infectious diseases and their presence is indicative of an immune response in consequence of an infection or contact with an immunogenic agent in general. Immunoglobulins themselves can be of detrimental effect as in the case of allergy, asthma or autoimmune diseases.

A typical example of an immunoglobulin test as marker for infection is a HIV antibody test. The presence of an antibody in the blood of the patient binding to HIV viral proteins is an indicator for HIV infection present in that patient. Even if the virus itself is not detectable in the patient, the presence of antibodies against HIV allows the conclusion that the patient is infected with HIV. Many different tests exist that detect immune globulins predominantly of class IgG or IgM directed against the antigen of the pathogens where the antigens used for the tests can be the pathogen as a whole or molecularly defined antigens from the pathogens such as proteins, oligosaccharides, lipids or nucleic acids. Antigens can also be a combination of those chemical entities such as glycolipids, glycosylated proteins or lipid proteins complexes.

In the case of allergy, the detection of allergen-specific immunoglobulin E (IgE) is a highly sensitive indicator for the clinical diagnosis of allergy. Here, the IgE itself is one of the key-players in the pathophysiology of the disease. For an allergen test the blood of the patient is examined for IgE molecules specifically recognizing the offending allergen. Aim of the test is then to determine if the patient sample has elevated IgE levels in general and/or if the sample contains IgE that reacts with a specific allergen. Typical examples for allergens are proteins from peanuts, celery, house dust mite, or birch pollen. In the case of autoimmune diseases the blood of the patient contains immunoglobulins reacting with antigens form the patient itself. Typical examples for autoimmune diseases are post-transfusion purpura with auto antibodies against platelet glycoproteins, rheumatoid arthritis, or lupus erythematodes. The examples above demonstrate the presence of antibodies in the case of disease, but a lack of immunoglobulins can also indicate a disease state such as for example the deficiency in plasma IgA as a very useful diagnostics test.

Whole blood, blood serum, blood plasma, blood cells, or parts of it are a source of testing material for diagnostics tests. Furthermore, other body fluids such as urine, semen, smears, cerebrospinal fluid, or saliva can be sources for suitable material to be used for *in-vitro* diagnostics tests (IVD).

Cancer biomarkers belong to the class of over-expressed or modified self antigens deriving from genetic modifications. A classical example thereof is canceroembryotic antigen (CEA) which is elevated in many types of cancer and prostate associated antigen (PSA).

Cytokines and chemokines belong to the class of small proteins and are over-expressed in basically all inflammatory diseases or after specific immunotherapy. Classical example is sepsis where pro-inflammatory cytokines are over-expressed long before the offending microorganism can be cultivated, or the detection of galactommannans and other products of fungal metabolisms in invasive mycoses. In these life-threatening diseases a fast and reliable diagnosis is required for saving lives. The current diagnostic methods for these diseases are too slow and in many cases the diagnosis for the patient comes too late.

There are many different assays on the market to perform biomarker assays. Each of these assay technologies is chosen for specific applications and analytes. Biochemical reagents are adapted accordingly for detecting and quantifying a specific biomarker. Out of the many methods to measure biomarkers, a commonly used technology for performing IVD tests is the Enzyme Linked Immuno Sorbent Assay (ELISA). ELISA is a heterogeneous assay format with a solid phase bound ligand and a liquid phase interacting with it. Another general aspect of all ELISA is the use of a solid substrate in the form of a pot called well with a volume of less than one milliliter having typically 300 microliter volume, in which the diagnostic biomarker assay of the patient sample is performed. ELISA wells are typically arranged in rows of 8 by 12 wells know as ELISA plate or microtiterplate with 96 individual wells. ELISA plates are made from organic polymers by injection molding, the typical materials are polystyrene but other organic polymers like PVC are also used.

All ELISA have the common measurement principle with at least one ligand present in the liquid phase and at least one analyte bound to the solid surface. The ligand in solution is coupled to an enzyme. The enzyme-labelled ligand in solution diffuses to the surface of the well and binds non-covalently to the surface-bound analyte. Typically this binding reaction takes about 30 to 120 minutes. Subsequently, the excess non-bound liquid ligand is removed by multiple washing steps by adding and removing a wash solution to the well. In a one-step ELISA the revelation of bound ligand - enzyme complex is performed by adding a color substrate and measuring color development. In a two-step ELISA, a secondary detection reagent is added and another incubation of 30 to 120 min is needed, followed by a second wash cycle, as described, after the first incubation and substrate addition. Some ELISA have been designed to include even a third ligand binding step for example an amplification or enhancing step, an example is the signal amplification with the Biotin-Avidin system before the substrate reaction is performed. Bound ligand is then detected by adding a suitable enzyme substrate. These enzyme substrates are colorless solutions of chemicals and the bound immobilized enzyme converts the colorless substrate to a colored product. The amount of color produced is dependent on the enzyme concentration and directly proportional to amount of enzyme bound to the immobilized analyte. The amount and concentration of colored product can now be read by suitable optical instruments quantitatively and is proportional to the amount of bound enzyme detector ligand conjugate and as such a quantitative measure for the analyte concentration. Other enzyme substrates generating luminescent or phosphorescent signal that can be measured are also used in combination with specific instrumentation needed for quantifying the output.

Other test systems used in IVD are bead based systems being in principle ELISA tests format with analytes immobilized on beads. Detector methods are then enzymatic or by fluorescence detection or alternatively by electrochemiluminescence. Many of these assays are performed using fully automated instrumentation reducing manual manipulation steps to perform an assay

Homogeneous assay formats using agglutination or homogeneous assays based on fluorescence resonance energy transmission FRET are other known assay technologies for *in-vitro* diagnostics.

The majority of test systems in use employ the basic principles of ELISA configurations with a need for multiple pipetting and washing steps to remove excess reagent carrying enzyme or fluorescent labels. Most methods require long incubation times and involve numerous manipulations of liquid transfer and washing steps, which are time consuming and demand in the diagnostic laboratory an automated solution.

More recent systems in comparison to ELISA and other immune assay technologies for the determination of biochemical and clinical analytes use biosensors. There are several technical solutions for biosensors available on the market.

A new technology for analysis of biomolecules is the evanescence field excitation of bound fluorophors and measures directly the binding events in real time as described in the patents EP1079226, EP1204856, EP1371967. The function principle of this evanescence field biosensor is the interaction of a surface-bound analyte with a ligand in solution by a non-covalent interaction. The ligand in solution is attached covalently to a fluorescing molecule. Preferred excitation of the fluorophoric molecule is with coherent light such as generated by e.g. a laser diode and then the emitted photons are detected. Suitable fluorophors for this are for example Cy5, Alexa dyes or the light-harvesting protein Allo-phycocyanine (APC).

The biochemical reaction takes place in a small well, similar to an ELISA well in form and dimensions with an additional optical entity on the bottom. As in ELISA the biosensor well and its optical bottom part are produced by injection molding of thermoplastic material, like e.g. polystyrene. Major differences between the ELISA method and the evanescence biosensor methods are the labels of the ligand in solution. For ELISA the label is an enzyme and for the evanescence technology the ligand is labelled with a fluorescing molecule. Measurement of the bound fluorophor, i.e. the fluorescing ligand in solution binding to the analyte immobilized on the evanescent surface, is achieved by specific excitation of the bound fluorescently labeled ligand using evanescence field excitation principle. Selective excitation of the bound fluorophor occurs by an evanescence field of light using an optical construction resulting in a specific excitation of an approximately 200 nm thick layer of liquid located at the bottom of the well and not exciting the fluorescently labeled ligand in excess present in the solution above the binding surface. Only fluorescently labeled ligands residing in the evanescent field are excited and emit photons. This method allows the observation of a binding reaction of the soluble ligand with the immobilized ligand in real time.

While most immune assays used in IVD testing have a long and tedious procedure involving incubation times, washes, addition of secondary or tertiary reagents or solutions to obtain a result for a biomarker, the evanescence biosensor allows to perform *in* vitro-diagnostic assay in short time. The complex and costly instrumentation of ELISA automates or for bead based immune assays are neither required nor necessary when using the evanescence biosensor for diagnostic applications.

However, no immuno-based assays are presently known that are both superior to ELISA in terms of test duration and ease of handling and at the same time provide comparable flexibility and specificity known from ELISA-based methods.

Accordingly, it is highly desirable to establish methods for detecting substances in various liquids, such as physiological liquids obtained from a patient, which are highly specific, sensitive, quickly deliver qualitative and quantitative results and are easy to use.

Therefore, the problem underlying the present invention is to provide novel methods which use the precise and highly versatile immune reactions e.g. employed in ELISA systems, while at the same time avoiding the above-mentioned disadvantages, such as prolonged test duration and the need of multiple and/or complicated manipulation steps to be carried out by the operator.

Accordingly, as one aspect of the present invention, there is provided a method for the detection of a substance in an aqueous, physiological or chemical liquid, comprising the steps: (i) providing a surface having at least a ligand L1 bound thereto, (ii) contacting the surface with a solution containing at least a substance to be detected and at least a fluorescently labelled ligand L2, wherein the substance to be detected and the fluorescently labelled ligand L2 may be the same, wherein either the substance to be detected, the fluorescently labelled ligand L2, or both interact with the surface-bound ligand L1 to form a complex comprising at least ligand L1, the substance to be detected and the fluorescently labelled ligand L2, (iii) exciting the surface-bound complex with an evanescence field of a light source, (iv) measuring the emitted fluorescence, and (v) determining a qualitative or quantitative result based on the emitted fluorescence, wherein said method does not require more than two liquid manipulation steps to obtain said result.

According to the present invention, the substance to be detected and ligand L2 are generally different from each other but may exceptionally be the same. In such cases, the solution contains at least a fluorescently labelled substance to be detected as both the substance to be detected and the fluorescently labelled ligand L2.

Herein, the term "detection" is not specifically restricted and does not only include the qualitative measurement of a substance of interest but expressly includes the quantitative determination thereof. In this context, "qualitative" as used herein means a determination of the presence or absence of a substance within a specific sample, while the term "quantitative" as used herein means a measurement of the content or amount of the substance to be detected within a specific sample.

In addition, the term "substance" is not specifically limited herein and includes any chemical or biochemical substance which may be of interest. Examples of said substances include biomolecules such as peptides, proteins, glycoproteins, nucleic acids, such as DNA and RNA, lipids, saccharides, hormones, gene products, and degradations products of tissues, as well as infectious agents/organisms such as cells, bacteria, fungi, mycoplasms, viruses, yeast, and parasites.

Further, the expression "aqueous, physiological or chemical liquid" used herein is not specifically restricted and includes all sorts of liquids, as long as said liquids may be usable in the method of the present invention without imparting the same. For example, the liquid may be an aqueous solution of the substance of interest, or may be a physiological liquid obtained or derived from a patient, such as whole blood, blood plasma, serum, synovial fluid, urine, saliva, or cerebrospinal fluid. The above expression "physiological liquid" further includes liquids derived from one or more patient tissues, e.g. obtained by biopsy, and further include whole cells or cell lysates of various types. "Chemical liquids" according to the present invention are liquids which include at least one solvent different from water, such as an alcohol, an ester, an aromatic solvent, an amine, etc.

According to the present invention, in step (i) of the method defined above, a surface is provided that has at least one ligand L1 bound thereto, i.e. immobilized thereon. Herein, the term "bound" preferably means that the ligand L1 is adhered to the surface by absorption (direct absorption). However, the ligand L1 may also be bound to the surface via a bridge element, for example a protein, such as an antibody or an antigen. The ligand L1 may also be bound to the surface by a covalent bond. This can be e.g. produced using an acrylate surface by conversion with a carbodiimide, for example. The term "bound" in the sense of the present invention includes adhesion to a surface or to another ligand, and includes both covalent and non-covalent interactions, like for example interactions based on ionic, polar or non-polar interactions.

Herein, ligand L1 can be placed on the surface by a common method. For example, a protein serving as ligand L1 can be coated on the surface. Ligand L1 may preferably be bound to the surface by absorption or by a covalent bond. After this step, the surface is preferably treated with another solution, and sites on the surface not adhered to ligand L1 are blocked or will be blocked, for example by another protein that basically does not react with the components contained in the solution to make contact. The above surface is the inside of a concave container, like a cuvette or a well of a microtiter plate or of a test cartridge, for example.

According to the present invention, the surface-bound ligand L1 can form a complex, wherein this complex at least includes, besides said ligand L1, the fluorescently labelled ligand L2 and the substance to be detected. With ligand L1 bound to the surface, the complex with the substance to be detected is "anchored" to the surface, i.e., fixed thereto and can at the same time be detected by marking it with a compound containing the fluorophor.

According to the present invention, a "complex" is understood to be a molecular coupling or bonding between two or more preferably chemical or biochemical substances. The complex is preferably formed by means of selective and/or specific conversions, especially preferred by antigen-antibody reactions. According to the invention, the term "conversion" includes both covalent and non-covalent interactions of two or more reaction partners, wherein both types of interaction can take place one after another within said complex. Non-covalent interaction can mean, for example, Van der Walls interactions, polar and/or ionic interaction of reaction partners. The term "reaction partner" means a compound with an affinity for another substance in this invention.

According to the present invention, the general mode of complex formation is not specifically restricted and includes, *inter alia,* the following examples:
(1) The substance to be detected has an affinity or a binding site for fluorescently labeled ligand L2. Accordingly, fluorescently labeled ligand L2 may bind covalently or non-covalently to the substance to be detected either before, after or at the same time the substance to be detected binds to ligand L1, thus forming a complex.
(2) The substance to be detected is ligand L2. In such a case, the substance to be detected itself is fluorescently labeled and is capable of forming a complex with surface-bound ligand L1. In such a case where the fluorescently labeled substance to be detected takes the function of ligand L2, said ligand L2 will not be required, since the substance to be detected itself carries the fluorophor.
(3) The substance to be detected contains fluorescently labeled ligand L2, i.e. ligand L2 is a part of the structure of the substance to be detected.
(4) Another compound contains fluorescently labeled ligand L2 or has an affinity to said fluorescently labeled ligand L2, wherein the other compound further contains at least one binding site for the substance to be detected. In this case, the other compound, the substance to be detected and ligand L2 can be in the solution as a conjugate or complex (all or only individually) or the conjugate is formed in the solution.

Moreover, the expression "liquid manipulation step" as used herein generally includes any step which transfers, removes or adds a liquid from and/or to a site of action. For example, liquid manipulation steps according to the present invention include adding, e.g. by pipetting, a liquid into the well of a cuvette, a microtiterplate or a test cartridge, or removing a liquid from such a well.

Herein, the term "light source" is not specifically restricted and includes any light source which is suited to create an evanescence field and excite the fluorophor bound to ligand L2 or the substance to be detected. For example, monochromatic light may be used as the light source. Light should be used that has a wavelength that preferably does not interfere with the emission of the fluorophor and preferably intersects with the absorption band of the dye. A laser is especially preferred as a light source, whose light emits a wavelength of at least 635 nm. In particular, if the supernatant solution is a serum, lasers that emit wavelengths from 600 to 700 nm are preferred, since the serum's inherent fluorescence is roughly 580 nm.

According to a further embodiment of the present invention, in the above-defined method, the solution of step (ii) further comprises at least one dye which absorbs in the absorption and/or emission range of the fluorophor.

According to the present invention, excitation of the fluorophor in the volume, i.e. of fluorophor not part of the surface-bound complex, can be suppressed if the solution to be placed in contact with the surface has at least one dye added to it that has an absorption in the absorption and/or emissions range of the fluorophor.

The absorption of the dye added to the volume is coordinated with the absorption and/or emission range of the fluorophor in the invention. One individual dye or a mixture of dyes can be used. The absorption range of the fluorophor generally correlates with the wavelength of the light source used. It is not necessary that the dye has an absorption maximum in this special range; a shoulder in the absorption spectrum can suffice. For example, if fluorophors like APC or Cy5 are used, the dye used can have an absorption between 600 nm and 700 nm, like for example Brilliant Blue FCF. The concentration of dye added depends on both the absorption coefficient of each dye in solution and the frequency of the light radiated. The concentration of dye can be adjusted, depending on the dye, so that the penetrating light can basically be absorbed within 1 mm above the surface.

According to a preferred embodiment, the above-defined method does not include any washing step.

In particular, the method of the present invention does preferably not require any steps of removing the volume containing excess reactants, such as fluorescently labelled ligand L2, and/or washing the surface on which ligand L1 and/or the complex to be measured are bound. This advantageously accelerates the method of the present invention to rapidly obtain the desired measurement results, avoids accumulation of waste liquids and operational errors when carrying out the method.

In a further embodiment of the present invention, the method as defined above is a one-step method. According to the present invention, "one-step" method means that only one complex-forming reaction takes place, which generally requires only one or at most two liquid manipulation steps to be conducted by the operator in order to obtain the desired measurement result. Thus, further reaction steps, such as the addition of more reagents after initial complex-formation are advantageously avoided.

Moreover, a further embodiment of the present invention relates to a method as defined above, wherein said method does not include liquid agitation.

In the present invention, the expression "liquid agitation" is not specifically limited and includes any type of agitation which occurs in the liquid, except for natural diffusion. For example, liquid agitation in the sense of the present invention includes shaking, stirring, ultrasonic agitation, etc. By avoiding any liquid agitation, except for natural occurring diffusion, the method can be carried out in a simple and effective manner, and thus avoids complicated technical setups including respective agitation devices.

According to a further embodiment of the above-defined method the result determined in step (v) is obtained within 10 minutes or less starting from the beginning of step (ii).

According to the present invention, the qualitative or quantitative result is obtained within 10 minutes or less after the solution containing at least the substance to be detected and the fluorescently labelled ligand L2 has been contacted with the surface to which the ligand L1 is bound. Preferably the result is obtained within 9 minutes or less, 8 minutes or less or 7 minutes or less. Further examples include periods of 6 minutes or less, 5 minutes or less or 4 minutes or less.

A further embodiment of the present invention relates to a method as defined above, wherein one or both of ligands L1 and L2 are independently selected from an antigen, an antibody or a fragment thereof.

According to a further embodiment, the present invention relates to a method as defined above, wherein the substance to be detected is a chemical or biomolecular substance selected from the group consisting of peptides, proteins, lipids, glycolipids, nucleic acids, toxins, hormones, chemokines, cytokins, immunoglobulins, antigens or auto antigens, as well as infectious agents including viruses, mycoplasms, bacteria, fungi, and yeasts, and fragments thereof.

In a further embodiment, the substance to be detected is an immunoglobulin selected from the group consisting of IgG, IgM, IgE, IgA and IgD.

Herein, the term "immunoglobulin", as well as the subtypes "IgG", "IgM", "IgE", "IgA" and "IgD" do not only refer to the respective immunoglobulins or subtypes as such, but also include fragments and derivatives thereof as long as these fragments and derivatives at least partly interact with the same compounds/structures when compared to the respective immunoglobulins or subtypes.

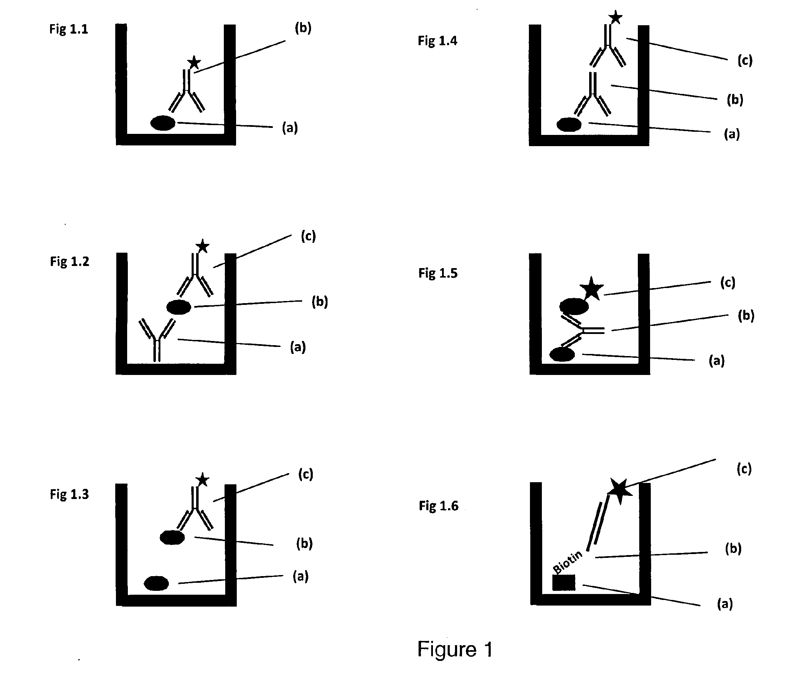

A further embodiment relates to the above-defined method, having the format of a simple binding assay, a sandwich assay, a competitive assay, or a double antigen assay. These formats are further described in Figure 1, as well as in the Examples.

All definitions provided above in respect to the method of the present invention also apply to the following aspects of the present invention, if not stated otherwise.

A further aspect of the present invention relates to a method for the detection of an allergy or an autoimmune disease against a specific allergen in a patient comprising the steps: (i) providing a surface having said specific allergen bound thereto, (ii) mixing a sample of a physiological liquid from the patient with a reaction mixture containing at least anti human IgE monoclonal antibodies, wherein said antibodies are covalently labelled with a fluorophor, (iii) contacting the surface with the solution obtained in step (ii) to form a complex comprising at least the allergen, IgE specific to said allergen and the fluorescently labelled anti human IgE monoclonal antibody, (iv) exciting the surface-bound complex with an evanescence field of a light source, (v) measuring the emitted fluorescence, and (vi) determining as a result, based on the emitted fluorescence, the amount of IgE in the sample within a time period of ten minutes or less starting from the beginning of step (ii), wherein said method requires step (ii) and (iii) as the only two liquid manipulation steps to obtain said result.

Another aspect of the present invention relates to a method for the detection of an infection with a specific pathogen in a patient comprising the steps: (i) providing a surface having an antigen of said pathogen bound thereto, (ii) mixing a sample of a physiological liquid from the patient with a reaction mixture containing at least an antigen of said pathogen, wherein said antigen is fluorescently labelled, (iii) contacting the surface with the solution obtained in step (ii) to form a complex comprising at least the immobilized antigen, at least one antibody specific to said antigen and the fluorescently labelled antigen, (iv) exciting the surface-bound complex with an evanescence field of a light source, (v) measuring the emitted fluorescence, and (vi) determining as a result, based on the emitted fluorescence, the amount of pathogen specific immunoglobulin in the sample within a time period of ten minutes or less starting from the beginning of step (ii), wherein said method requires step (ii) and (iii) as the only two liquid manipulation steps to obtain said result.

According to the present invention, the fluorescently labelled antigen present in the solution obtained in step (ii) of the above-defined method may be the same antigen as the one bound to the surface, or may be a different antigen, as long as it is capable of binding to the immunoglobulin to be detected.

A further aspect of the present invention relates to a method for the detection of a nucleic acid in a solution comprising the steps: (i) providing a surface having a nucleic acid complementary to the nucleic acid of interest bound thereto, (ii) contacting the surface with a sample of a solution potentially containing the nucleic acid of interest, wherein said nucleic acid of interest is fluorescently labelled, to form a complex comprising at least the complementary nucleic acid and the fluorescently labelled nucleic acid, (iii) exciting the surface-bound complex with an evanescence field of a light source, (iv) measuring the emitted fluorescence, and (v) determining as a result, based on the emitted fluorescence, the amount of nucleic acid of interest in the sample within a time period of ten minutes or less starting from the beginning of step (ii), wherein said method requires step (ii) as the only liquid manipulation step to obtain said result.

A further aspect of the present invention relates to a diagnostic test device for carrying out the methods as defined above.

According to the present invention, the diagnostic test device is not particularly limited and, for example, includes any test device which comprises an irradiation means for exciting the sample, a detector means capable of detecting the fluorescence obtained by said excitation, as well as a displaying means which displays the results to an operator. The diagnostic test device preferably contains a receptacle wherein the sample carrier, such as a cuvette, a microtiter plate or a test cartridge, is inserted

The cuvette, microtiter plate or test cartridge preferably contains glass or a plastic, especially preferably a plastic, such as polystyrene, polypropylene, polyethylene, polyethylene terephthalate, polycycloolefin, polyacrylnitrile, polymethylmethacrylate and/or mixtures and blends of these plastics. In principle, any plastic that basically absorbs no light in the visible range is suitable. In one embodiment, the plastic can also be dyed light blue, for example, in order to filter out an emission caused by scatter light. Plastic cuvettes, microtiter plates and test cartridges can be obtained inexpensively by injection molding and preferably have a reaction volume of 1 to 400 µl, and especially preferred 5 to 200 µl. Preferably, the cuvettes, microtiter plates or test cartridges of the present invention are made in one piece.

A further aspect of the present invention relates to a diagnostic test kit, comprising the diagnostic test device as defined above, at least one test cartridge providing the surface with ligand L1 bound thereto, and optionally one or more reaction compositions, diluents and/or auxiliary agents.

Herein, the expression "test cartridge" is not specifically restricted and includes any object which may be used to provide a surface on which ligand L1 is bound and on which may be contacted with the respective solutions or reactants. For example, the test cartridge may be a one-way disposable measurement cartridge that contains multiple, fully separated measurement wells, which are pre-conditioned to the specific measurement needs.

### The Figures show:

Figure 1 shows examples of assay formats that can be performed by using the method of the present invention.
   Figure 1.1 shows a simple binding assay. One ligand (a) is coated on the sensor surface and the fluorescing ligand (b) binds in a time dependent manner to the immobilized ligand (a). This is further demonstrated in Example 1 with coated Avidin and biotinylated APC as the detector reagent.
   Figure 1.2 shows a sandwich assay which is achieved by coating an antibody (a) to the sensor surface and having in the solution a fluorescently labelled second antibody (c) in solution, wherein both ligands (a) and (c) react independently and at the same time with the analyte (b). This is further demonstrated in Example 5, wherein the analyte protein IgE is detected with two monoclonal antibodies or in Example 11, which is a sandwich assay for the analyte beta HCG using two monoclonal antibodies.
   Figure 1.3 shows a competitive assay which is related to the simple binding assay with an immobilized ligand (a) and a fluorescently labelled ligand (c) in solution. A competitor (b) is binding to the ligand in solution (c). In Example 2 a competitive assay for biotin, also known as vitamin H, is described.
   Figure 1.4 shows a sandwich assay scheme for the detection of immune globulin. An immobilized ligand (a) is reacted with the immunoglobulin (b) and a second ligand (c) reacts with the immunoglobulin (b). In this case, ligand (c) is the fluorescently labelled ligand L2 in solution. In Example 3 a sandwich test detecting rAspf1-specific IgE in human serum is described.
   Figure 1.5 shows a double antigen sandwich antibody assay scheme comprising an immobilized antigen ligand (a) and a fluorescently labelled antigen ligand (c). The immunoglobulin (b) is the analyte in the sample. Immunoglobulins such as IgG are bi-functional molecules and have at least two binding sites for the antigen. Immunoglobulins such as IgM can react with 10 antigen molecules. This allows the formation of a double antigen sandwich assay also called a double antigen bridging assay for the detection of bi-functional immunoglobulins of all classes. Examples 6 and 7 describe tests for human anti-Toxoplasmose antibodies and for mouse anti-Ovalbumin IgG antibodies.
   Figure 1.6 shows an assay detecting DNA in a simple scheme with immobilized ligand (a) such as avidin, a biotinylated oligonucleotide (b) to bind the immobilized ligand avidin (a) and a fluorescently labelled oligonucleotide (c) the label is a Cy5 molecule. DNA detection tests are described in detail in Examples 10 and 11.
Figure 2 shows an avidin / biotinylated APC binding assay.
Figure 3 shows a competitive assay for biotin.
Figure 4 shows an assay for rAsp f1 binding human IgE.
Figure 5 shows a sandwich assay detecting total human IgE.
Figure 6 shows a one step double antigen antibody sandwich assay for toxoplasmose antibody.
Figure 7 shows a one step double antigen antibody sandwich assay for murine ovalbumin specific IgG.
Figure 8 shows an assay for nucleic acid, oligonucleotide detection.
Figure 9 shows an assay for nucleic acid, DNA detection with bound DNA fragment.
Figure 10 shows a sandwich assay for beta HCG.

The method for detecting substances in an aqueous, physiological or chemical liquid of the present invention advantageously combines the immuno-based technology of ELISA or other immuno-based technologies with an easy-to-use, quick and reliable evanescence field detection system. As a result, assaying methods are obtained which are highly versatile, specific and precise in analyzing the substances of interest in various liquids, but at the same time avoid the numerous manipulation steps (e.g. washing, blocking, etc.) required, for example, in ELISA. Moreover, advantageously, in the methods of the present invention no lengthy incubation periods are required, thus quickly delivering qualitative and quantitative results within 10 minutes or less.

The present invention will be further explained with reference to the examples below, without any limitation thereto.

### Examples

In the following, it is demonstrated how to convert immune assays and biochemical binding assays usually carried out in ELISA-systems into an evanescence biosensor assay. The adaptation of the biological components and reactants of an ELISA or bead based immunoassay is achieved for different assays and assay formats and can detect and measure accurately, rapidly, and quantitatively molecules with high sensitivity.

For the detection of proteins such as immunoglobulins or proteins a simple one-step sandwich assay format is possible. Essentially new is the very simple one-step assay procedure involving maximally two pipetting steps for the end user. Furthermore, to make the assays compatible for use as point-of-care (POC) testing or in the near-patient testing a major aspect was to eliminate any pretreatment step of the biological sample such as a dilutions, but use samples of plasma, serum, urine, or saliva in undiluted state. The majority of sandwich assays used to detect protein antigens and immune globulins in plasma and serum of patients are today two step assays.

The examples show the use of the evanescence biosensor technology for antibody sandwich assays to analyze and quantify of medium to high molecular weight analytes such as proteins. Other examples demonstrate the detection of antibodies by double antigen sandwich assays. Antibodies are directed against disease causing antigens, allergens, or infectious agents such as microorganisms. Surprisingly the assays work as a one-step assay showing high sensitivity and usability over a wide concentration range of antibody over three decades in concentration. It demonstrates the ability of the method to detect antigen-specific IgGs, other immunoglobulins such as IgM, IgA, IgE, and IgD in a simple fast one-step assay with high sensitivity, specificity, and over a large range of concentrations starting form tens of nanograms IgG per milliliter to hundreds of micrograms of IgG per milliliter. Importantly, the assays are performed without any washing step thereby reducing the workload to perform the tests. An additional advantage is the ability to detect high as well as low affinity antibodies. Detecting low affinity antibodies is of high importance as these antibodies can be washed off the immobilized antigen during the secondary incubation step and during the wash procedures of most immune assays and binding assays such as ELISA. This is of high diagnostic relevance when measuring immune responses directed against antigens of e.g. lipids, sugars, or glycolipids, and occasionally small peptides where binding affinities are low. Still, these low affinity immune responses are important in host defense or when early immune responses are of medical interest. A very prominent immune response with low affinity antibodies to sugar and sugar derivates are the blood group antigens A and B. Natural antibodies, of IgG and IgM subclass, are directed against saccharides and washing steps can remove these low affinity antibodies and generate false negative results in *in vitro* assays.

Sandwich assays for antigens and antibodies are often used today for the detection of proteins such as cardiac (Troponin, Myoglobin) and cerebral (S100) proteins for diagnosing heart attacks and stroke events. Further useful diagnostic applications are fertility hormone testing (beta HCG, LH, FSH etc.) and general protein and peptide hormone testing (TSH, insulin, cytokines, Interferons, PCT, BNP, ANP etc.). Antibody tests diagnosing infectious diseases are available on the market to practically all relevant infectious pathogens of viral, bacterial or other origin (HIV, HBV, HCV, Influenza, Toxoplasmose, *Aspergillus, Plasmodium, Streptococcus, E. coli* etc.). Other well-known antibody tests routinely done in diagnostics are allergy related diagnostic assays detecting quantitatively total and specific IgE and autoimmunity related assays that detect autoantibody binding to auto-antigens.

A second assay format commonly used in *in-vitro* diagnostic tests for low molecular weight compounds, where the sandwich format is technically not feasible, and used also for small peptides and occasionally for proteins occurring in high concentrations in plasma is the competitive immune assay format. Competitive assays have applications for testing small metabolites and drugs namely testing for drugs-of-abuse (Tetra-hydro-cannabinol, cocaine, opiates, amphetamines such as ecstasy, methadone, LSD, and Benzodiazepines amongst others), steroid hormones, metabolites such as histamine or tryptophan, and vitamins. Further tests in this format are antibiotics and pesticides and testing foods and food ingredients for these analytes represent relevant assays. Peptide hormones are also tested in a competitive assay format such as for example Vasopressin or Substance P. For some abundant analytes like C-reactive protein (CRP) the high concentration in normal human plasma of milligrams per milliliter poses problems to be measured reliably in a sandwich format. A solution can be pre-diluting the patient sample or another solution is to measure the analyte with a competitive assay to overcome the problem of high concentration of analyte and the high dose hook problem or prozone effect in the sandwich assay.

The third large group of analytes relevant in diagnostics is testing for nucleic acid DNA and RNA. This is done routinely by amplifying DNA or RNA using molecular biological amplification technologies (PCR). The detection of the amplified DNA or RNA from natural sources obtained without amplification step is demonstrated with the evanescence biosensor. Here, the evanescence based biosensor allows a fast and simple detection of nucleic acids labeled by suitable nucleic acid modifications introduced prior to the detection step. The basic principle is to label the nucleic acid to be analyzed specifically with a fluorescing moiety. This can be subsequently detected and quantified using the evanescence biosensor. The highly sensitive and highly specific detection of nucleic acids is achieved using a simple methodology similar to an ELISA format and yields a quantitative result in typically ten minutes or even less. As for the manipulation of DNA and RNA molecular biology the technologies use often denaturing conditions such as strong and denaturing detergents, chaotropic agents or heat, the use of enzyme/substrate combinations for the detection of nucleic acids is problematic. Here the evanescence biosensor is a compatible technology with most molecular biology methods as it uses no labile enzymes as detection label but instead relies on chemical fluorophors, which are stable to heat and other denaturing conditions. This enables new assay formats and applications in molecular biology that were not possible for ELISA formats with heat - and chemically labile enzymes and allows high sensitivity detection of DNA at low picomolar concentrations within five to ten minutes. With this high sensitivity and speed, nucleic acid detection can be performed even without amplification based on enzymatic amplification and gives a useful diagnostic tool for pathogen identification and quantitation. Yet another possibility for assay is a DNA protein binding assay, for example a promoter immune assay, where immobilized DNA is probed with a DNA-binding protein labelled with a fluorophor. In summary all ELISA assays can be converted to an evanescence biosensor assay.

### Example 1: Avidin/Biotin-APC Assay

A simple binding assay using the evanescence biosensor system is performed by reacting an avidin coated surface with various amounts of biotinylated allophycocyanin (bio-APC). An evanescence biosensor chip was coated with a solution of neutravidin in PBS. Coating is done by diluting the neutravidin stock solution to a 10 microgram per milliliter solution in PBS, adding 30 microliter of this solution to each well and incubating for 2 hours at room temperature. The coating solution is removed, the well is washed 3 times with PBS and finally 50 microliter of blocking solution are filled in the well. The blocking solution is a 1 % solution of BSA in PBS and contains 0.25 % Tween20. Blocking is for approximately 1 hour at room temperature and terminated by removing the blocking solution and adding the sample solution to be measured. Samples to be tested were bio-APC at various concentrations in a PBS buffer supplemented with 1% BSA, 0.05% Tween20 and 0.04% Brilliant Black BN. Each well is reacted with one specific concentration bio-APC.

Fluorescence is measured in real-time over 10 minutes using evanescence biosensor instruments and device described in EP1079226, EP1204856, EP1371967. The increase in measured photons is given a whole number and is the increase of counted photon within the first 10 minutes of the reaction for one specific well. The increase of photons measured in one well is given as the change of photons from time zero seconds to time 600 seconds as 'delta counts' (dc) with the measuring unit counts per second or abbreviated 'cps'. The photons emitting form one specific well are measured during the biochemical reaction at time intervals each measurement is for one second, but any value between 10 milliseconds or 20 seconds per measurement point can also be used. The measured photons are plotted in a graph on the Y-axis against the time on the X-axis and this procedure is performed for each well individually. Then a linear regression curve is generated by the software of the reader instrument and the average increase of photons per 10 minute observation time, is calculated automatically. A dc of 1532 counts per second, cps, the measured photons in one second, means for this specific well the number of photons measured has increased by 1532 cps within the 10 minutes of measurement.

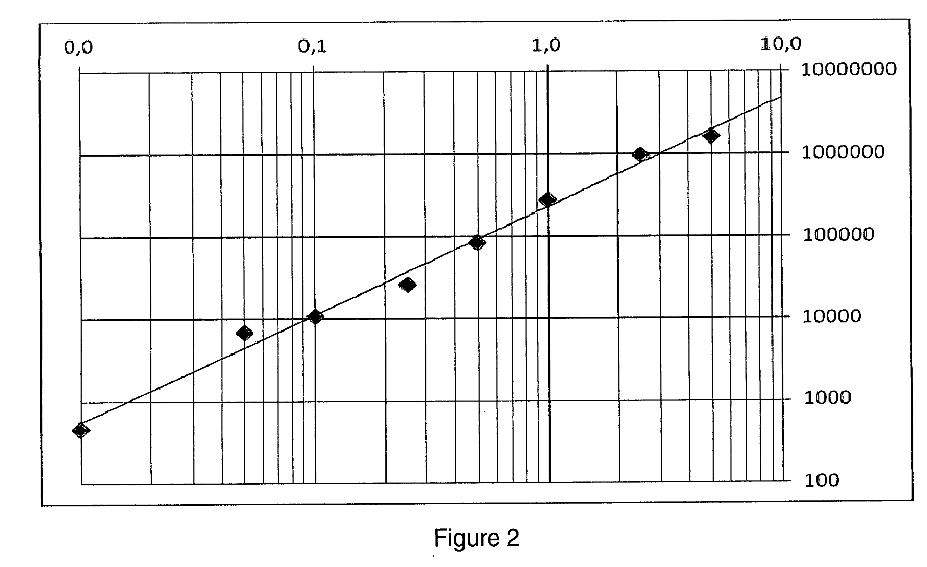

The increase in photon over time, dc in cps, is a now measure for the amount of analyte in the sample. The result shown in Figure 2 is obtained by plotting on the X-axis the concentrations of biotin APC used for the test in microgram per milliliter and on the Y-Axis the corresponding dc, the increase in photons during the first 10 min of the measurement. One observes a near linear relationship between the amount bio-APC used in the test and the observed signal using the evanescence biosensor. The detection limit for the bio-APC is less than 10 nanogram per ml.

### Example 2: Avidin/Biotin-APC Competitive Assay

A simple assay for the determination of biotin using the evanescence biosensor system is performed by reaction of an avidin coated biosensor surface prepared as described in example 1 first with a solution containing the biotin in various amounts in a buffer of 1%BSA, PBS, 0.05% Tween 20 for 1 hour at room temperature. After that the biotin solution is removed by washing the wells three times with PBS. Subsequently free biotin binding sites are detected with bio-APC.

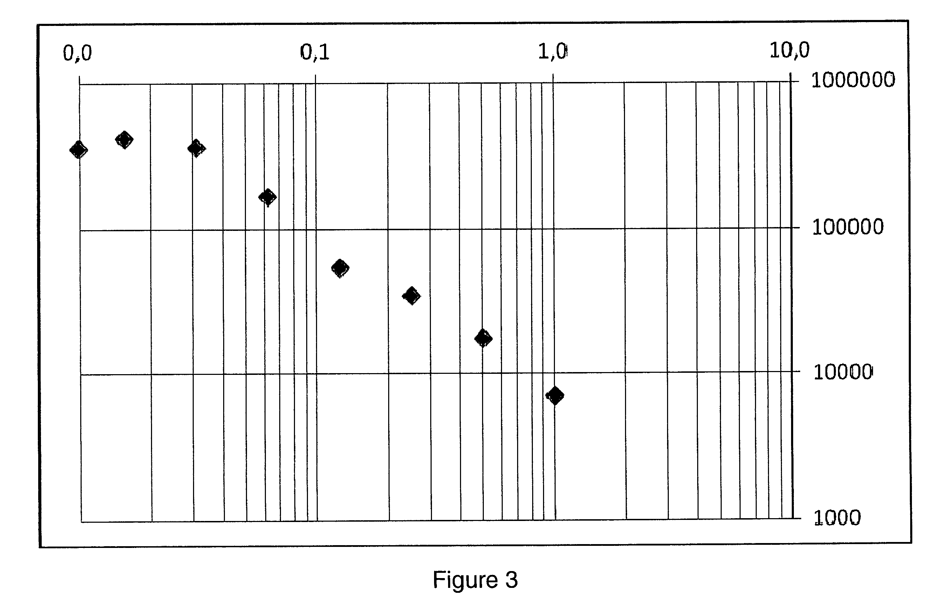

The increase in photon counts over time, dc in cps, is a now measure for the amount of free biotin in the sample. The result shown in Figure 3 is obtained by plotting on the x-axis the concentrations of free biotin used for the test in nmol per milliliter and on the Y-Axis the corresponding increasing cumulative photon count during the first 10 min of the measurement.

Observed is a typical competition curve, where increasing amounts of free biotin give a reduced signal in a dose dependent manner. Using this assay free biotin can be detected starting with the lowest detectable concentration of 50 pmol biotin per milliliter. The assay can also be performed as a one-step assay by mixing the biotin solution and the bio-APC outside of the well and analyzing the resulting mixture using an avidin biosensor chip. Competitive assays are useful for measuring small chemical and biochemical molecules such as steroids, antibiotic, drugs-of-abuse, pharmaceuticals, metabolites or vitamins. The measurement of Biotin, also known as Vitamin H, is one example of many different possibilities.

### Example 3: rASP f1-specific IgE Assay

The task to measure human IgE specific for rAsp f1 is solved by the following experimental design. rAsp f1 is one of the relevant major allergens of *Aspergillus fumigatus* and one of thousands of described allergens. A biosensor chip is coated with recombinant rAspf1 at 10 microgram per ml in PBS, according to the methods described in Example 1. rAsp f1 is expressed in E. *coli* and purified using standard chromatographic methods. For the measurement of human IgE antibodies in serum directed against rAspf1 four volume parts of human sera are mixed with one part of a fivefold concentrated reaction mixture. The fivefold concentrated mixture contains 5 mg/ml bovine IgG, 5% BSA, 0.2% Brilliant Black BN, 0.5% polyvinyl-pyrrolidon K70, 1% sucrose, 0.25% Tween20, 0.125% ascorbic acid, 10mM HEPES pH 7.4 in PBS and 50 microgram per milliliter of mouse anti human IgE monoclonal antibody, that has been covalently labelled with the APC fluorophor. 20 microliters of the mixture are pipetted to a rAsp f1 coated biosensor well and measured without delay in an evanescence biosensor instrument.

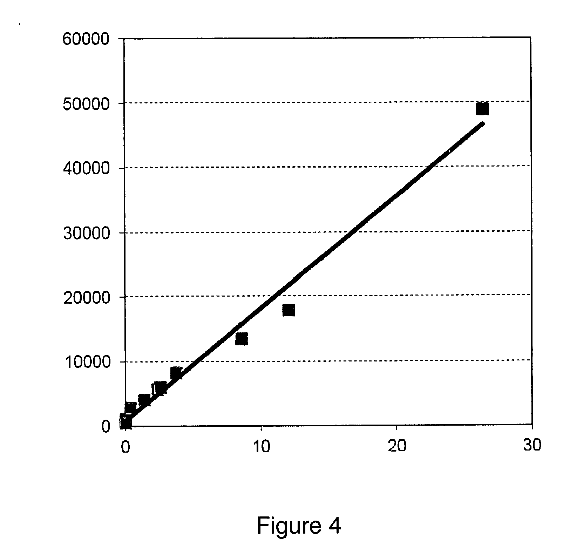

The increase in photons over time, dc in cps, is a now measure for the amount of anti rAsp f1 specific IgE present in the human serum sample. The result shown in Figure 4 is obtained by plotting on the X-axis the different know concentrations of anti rAspf1 specific human IgE present different serum samples given as CAP units kU/L and on the Y-Axis has the corresponding increase in photons during the first 10 min of the measurement. One observes a linear relationship between the amount rAsp f1 specific IgE in the serum samples and the signal dc in cps using the biosensor system. The correlation between the CAP units as determined by a Phadia rAsp f1 test and the evanescence biosensor system is 0.98 with a dc of 1738 cps per KU/L IgE. The detection limit for the evanescence biosensor test is below 1 kU/L. The evanescence biosensor allows a simple one-step determination of allergen specific IgE in whole human serum in 10 minutes with just two pipetting steps without any washing.

The example shown is one out of many tests that can be performed as by varying the antigen to be coated can be chosen from thousands of different antigens or allergens. Secondly, the format can also be used to detect other immunoglobulins from other subclasses for example immune globulins of isotype IgG, IgA, IgD, or IgM, and their corresponding sub-types.

### Example 4: Total IgE Assay

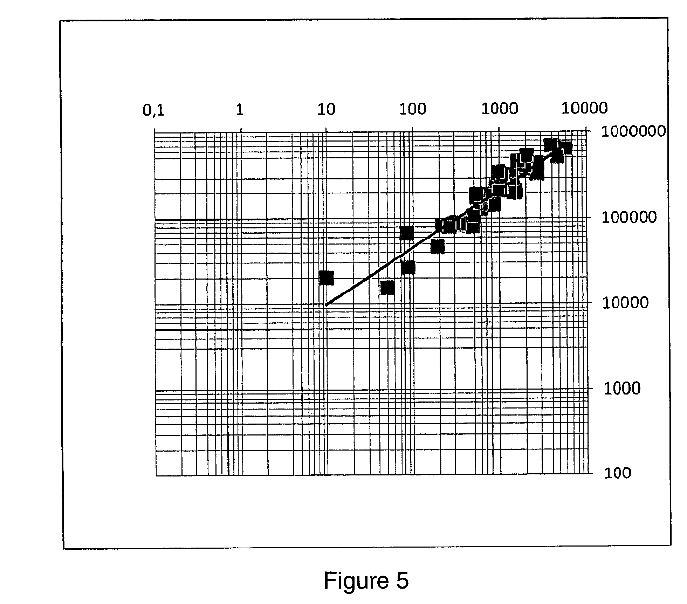

Measuring the concentration of total IgE, in serum is another frequently needed test in routine clinical diagnostics. For measuring IgE with the evanescence biosensor system the test design is a one-step sandwich assay. Biosensor chips are coated with and anti human IgE monoclonal antibody clone 7.3 at 10 microgram per milliliter and the detection mix for human IgE is prepared as described above. For this experiment, the mix is prepared as a threefold concentrated mix with 1.5 mg/ml bovine IgG, 3% BSA, 0.12% Brilliantblack BN, 0.15% polyvinyl-pyrrolidon K70, 0.3% sucrose, 0.15% Tween20, 0.125% ascorbic acid, 10mM HEPES pH 7.4 in PBS and 30 microgram per milliliter of mouse anti human IgE monoclonal antibody, that has been covalently labelled with APC. Human sera containing different amounts of IgE are diluted with PBS by mixing 1 part of human serum with 19 parts of PBS. 20 microliters of this mixture and 10 microliters of the threefold concentrated detection mix are mixed and 20 microliter of the resulting solution are pipetted to the coated biosensor well and the biosensor is measured directly in an evanescence biosensor instrument. The results are presented in Figure 5.

The increase in photon over time, dc in cps, is now a measure for the amount of total IgE present in the human serum sample. The result shown in Figure 5 is obtained by plotting on the X-axis the different concentrations of total human IgE present different serum samples given as CAP units kU/L and on the Y-Axis the corresponding increase in photons during the first 10 min of the measurement. One observes a linear relationship between IgE in the serum samples and the signal (dc in cps) using the biosensor system. The correlation between the CAP units as determined by a Phadia total IgE determination and the evanescence biosensor system is 0.89. The evanescence biosensor allows a simple one-step determination of total IgE in whole human serum in 10 minutes with just two pipetting steps and no washing steps. The methods allow similarly the detection of immune globulins of other subtypes.

### Example 5: Toxoplasmose antibody assay

A common test performed in *in vitro* diagnostics is to detect whether a patient has been infected with a pathogen by testing patient serum or plasma for antibodies reacting with the pathogen. Pathogens can be bacteria, virus, yeast, parasites or any other microbial pathogen. A positive anti-microbial antibody tests is in many cases indicative of an existing infection or a prior infection. This is done routinely in the blood bank setting, where all donors are screened serologically with antibody tests for HIV, HBV and HCV. Further routine screening tests are ToRCH panel of pathogens with four individual pathogens detected by serology tests. These pathogens are Toxoplasmose, Rubella, CMV and HSV.

A Toxoplasmose antibody test can be performed with the evanescence biosensor in a very simple and fast one-step assay as double-antigen-antibody assay. A double-antigen-antibody assay detects immune globulins reacting with the pathogen. The assay is not subclass specific, but instead all bi-functional immune-globulins give a positive reaction. The advantage of this assay format is that all immunoglobulins directed against the pathogen irrespective of immune subclass can be detected and this qualifies this setup as a screening test. In early phases of infection the predominant immune response is antibodies of IgM subtype and only later in the course of an infection an IgG response develops and dominates the immunoglobulin isotype spectrum. A double antigen assay allows the simultaneous detection of both IgG and IgM response and has a high diagnostic sensitivity. Furthermore, as the evanescence biosensor assay does not involve washing steps during the assay procedure and uses undiluted serum one can detect antibodies having only low affinity antibodies, which are difficult to detect in ELISA. Low affinity antibodies are washed away in ELISA procedures during the washing steps and during the normal incubation periods for example as during the incubation period with a secondary antibody enzyme conjugate. Examples for low affinity antibodies are antibodies in very early phases of infections or antibodies directed against small low molecular weight antigens such as saccharide and lipid antigens of bacteria and other pathogens, where antibodies due to their low affinity are sometimes difficult to detect in the sera of infected animals and humans when classical ELISA methods are used. Other sugar antigens where the biosensor technology can be used advantageously are the blood group antigens A and B and their low affinity antibodies directed against these blood groups antigens.

Toxoplasmose antigen is prepared as particulate matter from tissue culture supernatant and purified according to standard procedures. One aliquot of this antigen is coated on a biosensor chip by absorption essentially as described for Avidin. Another aliquot is fluorescently labeled with APC. One step double antigen tests are performed by mixing 20 microliter anti-Toxoplasmose IgG standards and 10 microliter of a threefold concentrated reaction mix consisting of Tris buffered 1% Casein, 2% BSA, 0.3% Tween 20 with an essentially neutral pH and containing 60 microgram per milliliter of Toxoplasmose antigen APC conjugate. Immediately after mixing, 20 microliters of the resulting solution are transferred to a Toxoplasmose antigen coated biosensor well and the biosensor device is measured without delay in an evanescence biosensor instrument. The increase in photon over time, (dc in cps), is a now a measure for the amount of anti Toxoplasmose specific immunoglobulin.

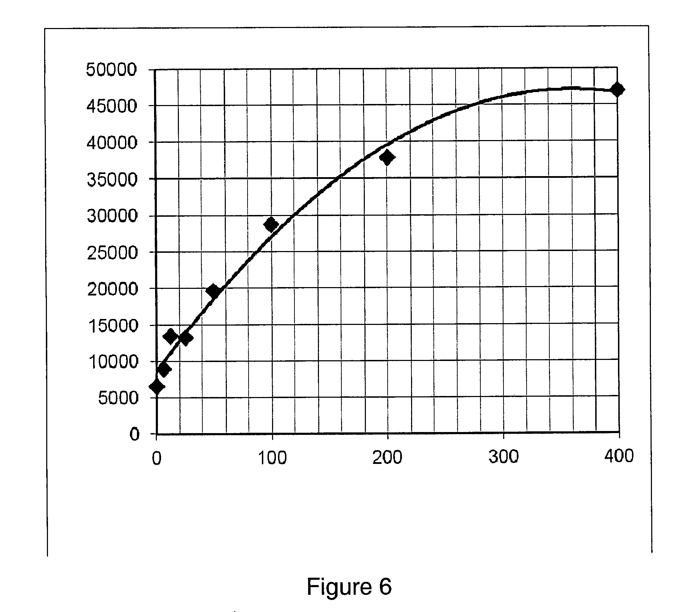

The result is shown in Figure 6 by plotting on the X-axis the different concentrations of anti-Toxplasmose standard given in IU/mL and on the Y-Axis the corresponding increase in photons during the first 10 min of the measurement. One observes a relationship between the amount of anti-Toxplasmose standard in the sample tested and the evanescence biosensor signal. The correlation of signal to given units in the standard after curve fitting is 0.98 and the detection limit is below 10 IU/mL of the standard preparation, the assay has an equal or better performance than an ELISA.

### Example 6: Ovalbumin antibody assay

In allergy research mice are immunized with minute amounts of Ovalbumin, to generate an anti-Ovalbumin IgE response *in vivo.* The Ovalbumin immunization leads in the experimental animals also to a strong anti-Ovalbumin IgG response and this can be measured. The IgG fraction of immunized mice was Protein G purified and the anti-Ovalbumin IgG concentration determined by an ELISA. This mouse IgG anti-ovalbumin preparation was subsequently tested in a one-step evanescence biosensor assay.

The assay format is a double antigen sandwich antibody assay, made by immobilizing Ovalbumin on the sensor surface by incubating a 10 microgram per milliliter Ovalbumin solution in PBS overnight in the well, followed by washing and blocking steps as described in Example 1. For the detection, 20 microliters of a solution containing defined amounts of anti-Ovalbumin mouse IgG were mixed with 10 microliter of a solution containing Ovalbumin labelled with APC at 60 microgram per ml in a buffer of 3% BSA, 0.12% Brilliant Black BN 0.3% polyvinyl-pyrolidon K90, 0.15% Tween20, 0.075 % ascorbic acid, 6mM HEPES pH 7.4, 2mM P04 pH 7.4 and 30mM NaCl. 20 microliters of this mixture were analyzed immediately without unnecessary delay on the Ovalbumin coated biosensor chip and the biosensor reader.

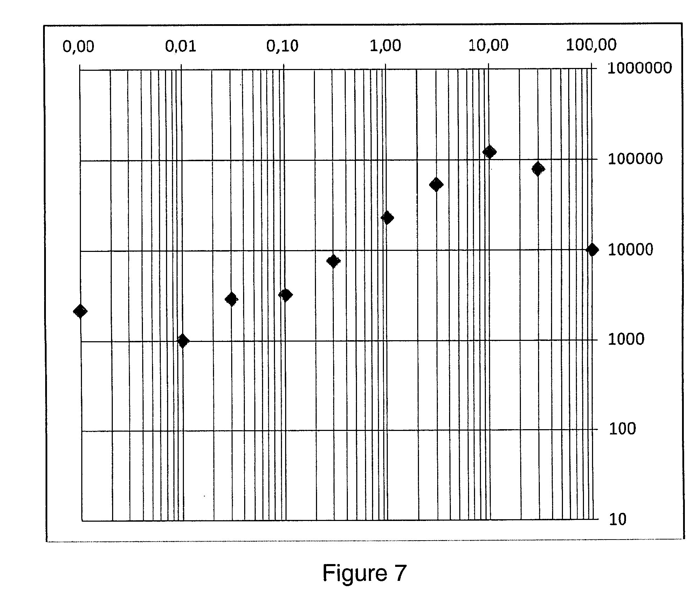

The increase in photon over time (dc in cps) is now a measure for the amount of mouse anti-Ovalbumin IgG in the original sample. The result shown in Figure 7 is obtained by plotting on the X-axis the different concentrations of anti-Ovalbumin IgG given in units of microgram per milliliter and on the Y-Axis the corresponding increase in photons during the first 10 min of the measurement. One observes a relationship between the amount of anti-Ovalbumin IgG in the test in the range for 30 nanogram IgG per milliliter to 10 microgram per milliliter. Higher amounts of IgG at physiologic irrelevant concentrations over 30 microgram per milliliter of antigen specific IgG result in a reduced measure signal. Lower than expected form linearity. This is due to the high dose hook (prozone) effect reducing the measured signal. Nevertheless all samples from 30 nanogram IgG per milliter to 100 microgram per milliliter give a positive signal. This demonstrates the use of a one-step double antigen sandwich antibody assay for detecting IgG and other immune globulins as diagnostic tool for in pathogen detection. It is also obvious that using a sandwich antigen assay it is possible to do a pathogen antigen detection examples are the HIV p24 or the HBV S antigen or the HBS c antigen or malarial antigens.

### Example 7: Assay for nucleic acid, oligonucleotide detection

Detecting nucleic acids such as DNA or RNA is another common task in diagnostics and R&D. A simple assay for DNA is done by coating an evanescence biosensor chip with avidin as described in Example 1. After coating and blocking, the chips were washed 3 times with PBS, 3 times with a 1% sucrose solution and then all liquid removed by tapping the biosensor device dry on towel paper. The resulting chips are left for 2 hours at ambient temperature to achieve drying. Biosensors prepared with this washing procedure have a performance identical to biosensor chips in non-dried format. For the tests, a solution containing 40 pmol per milliliter of the 3' biotinylated oligonucleotide, 5'-ACT GGC GAA CTA CTT ACT CT -3'-BIO, in a buffer consisting of 4 times concentrated SSC, 0.1% SDS and 0.04% Brilliant Black BN and various amounts of complementary 5' Cy5 labelled complementary analyte oligonucleotide, Cy5-5'-AGA GTA AGT AGT TCG CCA GT -3', were mixed at room temperature and immediately after mixing 20 microliters of the mixture were analyzed by adding into an avidin-coated well and read with the evanescence reader instrument for 5 minutes. The Cy5-labelled oligonucleotide concentration was varied over a range from 0 pmol per ml to 1000 pmol per ml.

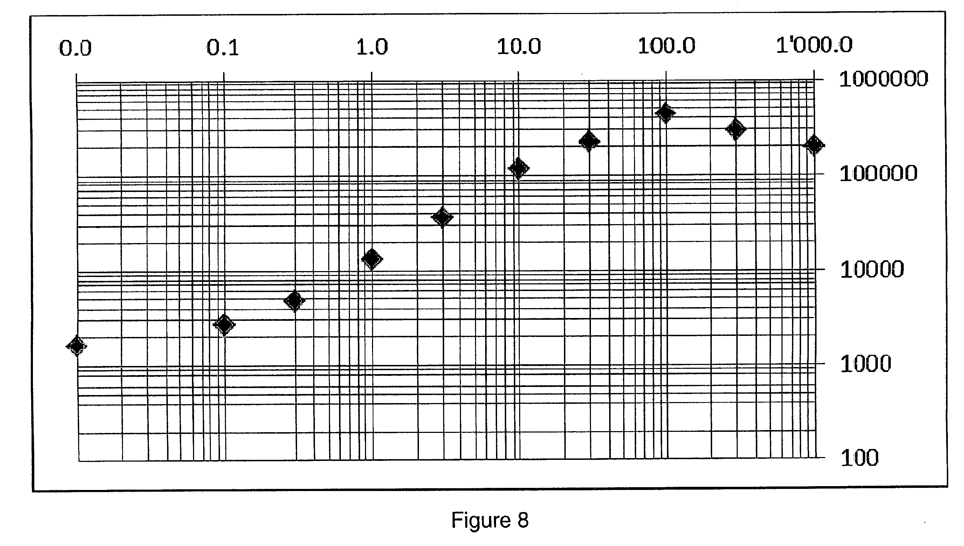

The increase in photon over time, dc in cps, is now a measure for the amount of Cy5 labelled oligonucleotide. The result shown in Figure 8 is obtained by plotting on the X-axis the different concentrations of Cy5 oligonucleotide in pmol per milliliter and on the Y-Axis the dose dependent increase in photons during the first 5 min of the measurement with increasing amounts of analyte Cy5 oligonucleotide for Cy5 oligo concentrations in the test from 0 to 100 pmol per milliliter. The analytical sensitivity is under 0.1 pmol per milliliter for the quantitative detection of DNA in a 5 minute assay.

### Example 8: Assay for nucleic acid, DNA detection with bound DNA fragment.

Another assay format for detecting nucleic acids such as DNA or RNA is by immobilizing an oligonucleotide on the sensor surface, hybridizing with a soluble complementary Cy5-labelled DNA fragment and observing the reaction in a time-resolved manner with the evanescence biosensor. A simple assay for DNA is done by coating an evanescence biosensor chip with avidin as described in Examples 1 or 7. After the blocking step, the biosensor is incubated with the a solution containing 40 pmol per milliliter of the 3' biotinylated oligonucleotide, 5'-ACT GGC GAA CTA CTT ACT CT -3'-BIO, in a buffer consisting of 4 times concentrated SSC, 0.1% SDS and 0.04% Brilliant Black BN for 2 hours at room temperature. Excess non-reacted biotinylated oligonucleotide is washed away by multiple washes before the test.

For the tests various amounts of the 5' Cy5-labelled analyte oligonucleotide, Cy5-5'-AGA GTA AGT AGT TCG CCA GT-3', were prepared in a buffer consisting of 4 times concentrated SSC, 0.1 % SDS and 0.04% Brilliant Black BN. Of this solution 20 microliters were added to the evanescence biosensor well and read without delay in the evanescence reader instrument for 5 minutes. The Cy5-labelled oligonucleotide concentration was varied over a range from 0 pmol per ml to 100 pmol per ml.

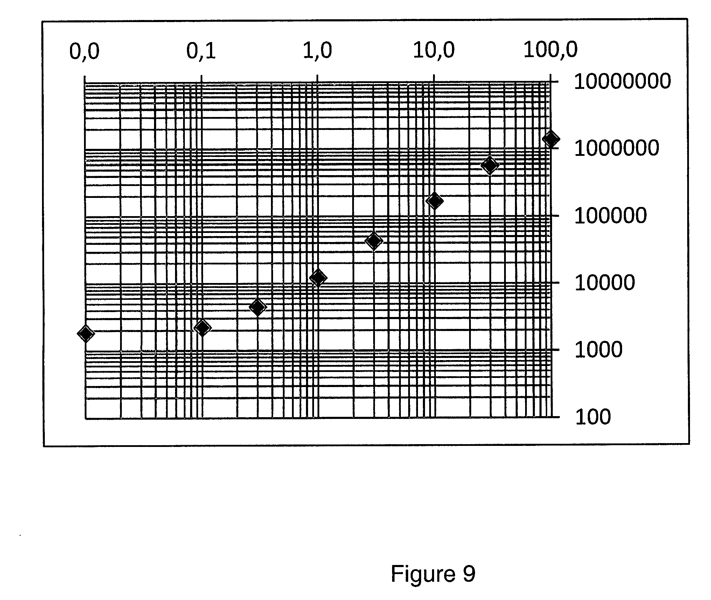

The increase in photon over time (dc in cps) is a now measured for the amount of Cy5-labelled oligonucleotide. The result shown in Figure 9 is obtained by plotting on the X-axis the different concentrations of Cy5 oligonucleotide in pmol per milliliter and on the Y-Axis the corresponding increase in photons during the first 5 min of the measurement. One observes a relationship between the Cy5 oligo in the test form 0.2 to 100 pmol per milliliter. The analytical sensitivity is at 0.3 pmol per milliliter or less to detect DNA in a 5 minute assay quantitatively.

### Example 9: Sandwich assay detecting beta HCG

Another common assay format is a sandwich assay for the detection of a protein molecule. A typical example for a sandwich assay is a test for the pregnancy hormone human beta chorionic gonadotropin. The test is produced by immobilizing an antibody to beta HCG on the evanescence biosensor surface by incubating a 10 microgram per milliliter anti-beta HCG monoclonal antibody, Medix 5016, solution in PBS overnight, followed by washing and blocking steps as outlined in Example 1. For the detection 20 microliters of a solution containing beta HCG in defined amounts, Sigma C0434, were mixed with 10 microliter of a solution containing another monoclonal antibody to beta HCG, Medix 5501, covalently labeled with APC, at 30 microgram per ml in a buffer of 3mg/ml bovine IgG, 3% BSA, 0.12% Brilliant Black BN 0.3% polyvinyl-pyrolidon K90, 0.6% sucrose, 0.15% Tween20, 0.075 % ascorbic acid, 6mM HEPES pH 7.4, 2mM P04 pH 7.4 and 30mM NaCl. 20 microliters of this mixture were analyzed immediately without unnecessary delay on the coated dry biosensor chip using the biosensor reader.

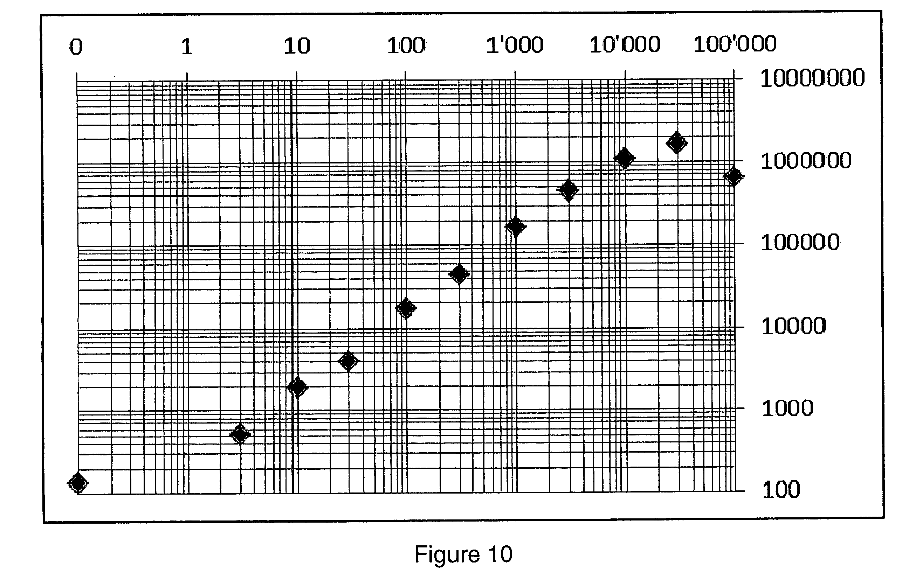

The increase in photon over time, (dc in cps), is a now measure for the amount of beta HCG in the sample. The result shown in Figure 10 is obtained by plotting on the X-axis the different concentrations of beta HCG given in International Units per liter and on the Y-Axis the corresponding increase in photons during the first 10 min of the measurement. One observes a linear relationship between the diluted amounts of HCG in the test in the range for 10 U/L to 30000 U/L. Higher amounts of analyte result in a reduced measured signal, lower than expected form linearity. This is due to the high dose hook effect or prozone effect reducing the measured signal. Nevertheless all samples from 5U/L to 30000U/L of beta HCG are measured quantitatively and the observed curve has a sigmoidal shape as the theory for an immune assay requires. This demonstrates the usability of the evanescence biosensor to measure in sandwich assays biochemical analytes with high sensitivity and high specificity and with a quality as good as other commonly used immunoassays such as ELISA, beads technologies or other immune assay or binding assay formats.

## Claims

1. A method for the detection of a substance in an aqueous, physiological or chemical liquid, comprising the steps:
(i) providing a surface having at least a ligand L1 bound thereto,
(ii) contacting the surface with a solution containing at least a substance to be detected and at least a fluorescently labelled ligand L2, wherein the substance to be detected and the fluorescently labelled ligand L2 may be the same,
wherein either the substance to be detected, the fluorescently labelled ligand L2, or both interact with the surface-bound ligand L1 to form a complex comprising at least ligand L1, the substance to be detected and the fluorescently labelled ligand L2,
(iii) exciting the surface-bound complex with an evanescence field of a light source,
(iv) measuring the emitted fluorescence, and
(v) determining a qualitative or quantitative result based on the emitted fluorescence,
wherein said method does not require more than two liquid manipulation steps to obtain said result.

2. The method according to claim 1, wherein the solution of step (ii) further comprises at least one dye which absorbs in the absorption and/or emission range of the fluorophor.

3. The method according to claim 1 or 2, wherein the method does not include any washing step.

4. The method according to any one of claims 1 to 3, wherein the method is a one-step method.

5. The method according to any one of claims 1 to 4, wherein the method does not include liquid agitation.

6. The method according to any one of claims 1 to 5, wherein the result determined in step (v) is obtained within 10 minutes or less starting from the beginning of step (ii).

7. The method according to any one of claims 1 to 6, wherein one or both of ligands L1 and L2 are independently selected from an antigen, and antibody or a fragment thereof.

8. The method according to any one of claims 1 to 7, wherein the substance to be detected is a chemical or biomolecular substance selected from the group consisting of peptides, proteins, lipids, glycolipids, nucleic acids, toxins, hormones, chemokines, cytokins, immunoglobulins, antigens or auto antigens, as well as infectious agents including viruses, mycoplasms, bacteria, fungi, yeasts, and fragments thereof.

9. The method according to claim 8, wherein the substance to be detected is an immunoglobulin selected from the group consisting of IgG, IgM, IgE, IgA and IgD.

10. The method according to any one of claims 1 to 9, which has the format of a simple binding assay, a sandwich assay, a competitive assay, or a double antigen assay.

11. A method for the detection of an allergy or an autoimmune disease against a specific allergen in a patient comprising the steps:
(i) providing a surface having said specific allergen bound thereto,
(ii) mixing a sample of a physiological liquid from the patient with a reaction mixture containing at least anti human IgE monoclonal antibodies, wherein said antibodies are covalently labelled with a fluorophor,
(iii) contacting the surface with the solution obtained in step (ii) to form a complex comprising at least the allergen, IgE specific to said allergen and the fluorescently labelled anti human IgE monoclonal antibody,
(iv) exciting the surface-bound complex with an evanescence field of a light source,
(v) measuring the emitted fluorescence, and
(vi) determining as a result, based on the emitted fluorescence, the amount of IgE in the sample within a time period of ten minutes or less starting from the beginning of step (ii),
wherein said method requires step (ii) and (iii) as the only two liquid manipulation steps to obtain said result

12. A method for the detection of an infection with a specific pathogen in a patient comprising the steps:
(i) providing a surface having an antigen of said pathogen bound thereto,
(ii) mixing a sample of a physiological liquid from the patient with a reaction mixture containing at least an antigen of said pathogen, wherein said antigen is fluorescently labelled,
(iii) contacting the surface with the solution obtained in step (ii) to form a complex comprising at least the immobilized antigen, at least one antibody specific to said antigen and the fluorescently labelled antigen,
(iv) exciting the surface-bound complex with an evanescence field of a light source,
(v) measuring the emitted fluorescence, and
(vi) determining as a result, based on the emitted fluorescence, the amount of pathogen specific immunoglobulin in the sample within a time period of ten minutes or less starting from the beginning of step (ii),
wherein said method requires step (ii) and (iii) as the only two liquid manipulation steps to obtain said result.

13. A method for the detection of a nucleic acid in a solution comprising the steps:
(i) providing a surface having a nucleic acid complementary to the nucleic acid of interest bound thereto,
(ii) contacting the surface with a sample of a solution potentially containing the nucleic acid of interest, wherein said nucleic acid of interest is fluorescently labelled, to form a complex comprising at least the complementary nucleic acid and the fluorescently labelled nucleic acid,
(iii) exciting the surface-bound complex with an evanescence field of a light source,
(iv) measuring the emitted fluorescence, and
(v) determining as a result, based on the emitted fluorescence, the amount of nucleic acid of interest in the sample within a time period of ten minutes or less starting from the beginning of step (ii),
wherein said method requires step (ii) as the only liquid manipulation step to obtain said result.

14. A diagnostic test device for carrying out the method according to any one of claims 1 to 13.

15. A diagnostic test kit comprising the diagnostic test device according to claim 14, at least one test cartridge providing the surface with ligand L1 bound thereto, and optionally one or more reaction compositions, diluents and/or auxiliary agents.


SEQUENCE LISTING

<110>  Davos Diagnostics
       Schawaller, Manfred  Dr.
       Rhyner, Claudio
       Akdis, Cezmi
       Wiki, Max
       Crameri, Reto

<120>  Real Time Diagnostic Assays Using An Evanescence Biosensor

<130>  D 3442EU - py

<160>  2

<170>  PatentIn version 3.5

<210>  1
<211>  20
<212>  DNA
<213>  Artificial

<220>
<223>  Test Sequence of Examples 7 and 8

<400>  1
actggcgaac tacttactct                                                20

<210>  2
<211>  20
<212>  DNA
<213>  Artificial

<220>
<223>  Test Sequence of Examples 7 and 8

<400>  2
agagtaagta gttcgccagt                                                20